# EUROPEAN PATENT SPECIFICATION

(11) **EP 0 920 870 B1**
(45) Date of publication and mention of the grant of the patent: **06.11.2002**
(21) Application number: 98103505.8
(22) Date of filing: 27.02.1998
(51) Int. Cl.: A61K 31/70, A61K 31/35, A61P 3/00

(54) **Flavanone-containing composition**
Flavanon-enthaltende Zusammensetzungen
Compositions contenant des flavanones

(30) Priority: 08.12.1997 JP 35198997
(43) Date of publication of application: 09.06.1999
(73) Proprietor: National Agricultural Research Organization, Ibaraki (JP)
(72) Inventor: Sekiya, Keizo, Zentsuji-shi, Kagawa-ken (JP)
(74) Representative: VOSSIUS & PARTNER

(56) References cited:
- DATABASE WPI Section Ch, Week 9739 Derwent Publications Ltd., London, GB; Class D13, AN 97-419359 XP002066948 & JP 09 187 230 A (NIPPON FLOUR MILLS CO LTD) , 22 July 1997
- DATABASE WPI Section Ch, Week 9702 Derwent Publications Ltd., London, GB; Class B02, AN 97-017298 XP002066949 & JP 08 283 154 A (NIPPON SHINYAKU CO LTD) , 29 October 1996
- DATABASE WPI Section Ch, Week 9732 Derwent Publications Ltd., London, GB; Class B02, AN 97-347392 XP002066950 & JP 09 143 070 A (NIPPON FLOUR MILLS CO LTD) , 3 June 1997
- DATABASE WPI Section Ch, Week 9241 Derwent Publications Ltd., London, GB; Class A96, AN 92-337587 XP002066951 & JP 04 243 822 A (TSUMURA & CO) , 31 August 1992
- CHOI J.S., YOKOZAWA T. ET AL.: "Antihyperlipidemic effect of flavonoids from prunus davidiana" JOURNAL OF NATURAL PRODUCTS, vol. 54, no. 1, January 1991 - February 1991, pages 218-224, XP002066943
- GALATI E.M., TROVATO A., ET AL.: "Biological effects of hesperidin, a citrus flavonoid. (note III): Antihypertensive and diuretic activity in rat" FARMACO, vol. 51, no. 3, March 1996, pages 219-221, XP002066944
- MONFORTE M.T., TROVATE A.. ET AL.: "Biological effects of hesperidin, a citrus flavonoid. (note II): hypolipidemic activity on experimental hypercholesterolemia in rat." FARMACO, vol. 50, no. 9, September 1995, pages 595-599, XP002066945
- RATHI A.B., ET AL.: "Action of vitamin P like compounds on lysosomal status in hypercholesterolemic rats" ACTO VITAMINOL. ENZYMOL., vol. 5, no. 4, 1983, pages 255-261, XP002066946
- CHOI J.S., ET AL.: "Improvement of hyerglycemia and hyperlipemia in streptozotocin diabetic rats by a methanolic extract of prunus davidiana stems and its main component, prunin" PLANTA MEDICA, vol. 57, no. 3, June 1991, pages 208-211, XP002066947

## Description

The present invention relates to a composition comprising flavanone and insulin. Specifically, the present invention relates to a composition for accelerating cell differentiation into a fat cell. More specifically, the present invention relates to a composition that acts on a precursor fat cell to accelerate differentiation into a fat cell showing a activity of decreasing blood sugar and/or lipid level.

A fat cell has an active function in metabolism, mainly lipid metabolism, in vivo. A fat cell is formed when a specific induction factor (substance having an activity of controlling differentiation) acts on a precursor fat cell.

In a precursor fat cell, the ability to metabolize sugar or lipid is notably weak. However, when this precursor fat cell is differentiated into a fat cell, the ability to metabolize sugar or lipid is increased, and sensitivity to a hormone such as insulin or the like is also acquired.

It is already known that growth hormone, insulin and the like which are in vivo hormones expedite differentiation into a fat cell.

A substance that induces the differentiation of a precursor fat cell into a fat cell acts in vivo effectively on a hormone such as insulin and the like to be able to activate the metabolism of a sugar or a lipid and decrease sugar or lipid level in the blood. Accordingly, such a substance to induce differentiation into a fat cell attracts interest as a new agent for preventing or treating various diseases such as adult diseases, especially, diabetes, hyperlipemia and the like.

As stated above, the metabolism, mainly sugar or lipid metabolism, is actively conducted in the fat cells. However, when the metabolic function fails owing to the irregular eating habits or the like, this may cause the development of various diseases, such as diabetes, hyperlipemia, hypertension, gout, ischemic heart disease, fatty liver, cholelithiasis, menstrual disorder, sterility and the like.

As the substance that accelerates cell differentiation into a fat cell, pioglitazone has been reported other than the above-mentioned hormones (Sandouk. T. et al., American Journal of Physiology, 33, C1600 - C1608, 1993). According to this report, it is considered that when the differentiation of a precursor fat cell into a fat cell is accelerated, the insulin activity in the periphery of the fat tissue or the like is increased, thereby decreasing the blood sugar level.

Besides, it is known that clofibrate exhibits the same activity as a hyperlipemia treating agent (P. Verrando et al., Biochemica et Biophysica Acta, 663, 255 - 265, 1981, R. Brandes et al., Biochimica et Biophysica Acta, 877, 314 - 321, 1986, R. Brandes et al., Life Sciences, 40, 935 - 941, 1987).

However, these synthetic compounds are likely to induce side effects that have an adverse effect on the human body, and they are extremely problematic in the practical use.

Thus, the technical problem underlying the present invention was to provide substances that accelerate differentiation of a precursor fat cell into a fat cell without causing harmful side effects on the human body.

The solution to said technical problem is solved by the embodiments characterized in the claims.

According to the present invention it was found that substances which accelerate the differentiation are present in ingredients of herb medicines, and that β-carotene which is an extract of a banana or a carrot and isoflavones contained in soybeans have the same activity of accelerating or inhibiting cell differentiation as the in vivo hormones.

It is an object of the present invention to develop a substance that exhibits an activity of accelerating the differentiation into a fat cell from natural substances which are completely safe.

It was found that an extract of plants such as citrus fruits has the above-mentioned activity, and that its active substance is flavanones.

Accordingly, the present invention relates to a composition for accelerating cell differentiation into a fat cell comprising a flavanone and insulin in an amount effective for accelerating cell differentiation into a fat cell.

In a preferred embodiment the composition of the present invention is a food, food material or animal feed.

In a further embodiment the present invention relates to a pharmaceutical composition comprising a flavanone and, optionally, a pharmaceutically acceptable carrier.

In a preferred embodiment the flavanone comprised in the composition or the pharmaceutical composition of the present invention is at least one compound selected from the group consisting of naringenin, naringenin-7-glycoside, naringin, hesperetin and hesperidin.

Furthermore, the present invention relates to the use of a flavanone for the preparation of a composition for accelerating cell differentiation into a fat cell, for decreasing blood sugar or lipid level or for treating or preventing diabetes, hyperlipemia, hypertension, gout, ischemic heart disease, fatty liver, cholelithiasis, menstrual disorders or sterility.

Preferably, said flavanone used for the preparation of the composition of the invention is at least one compound selected from the group consisting of naringenin, naringenin-7-glycoside, naringin, hesperetin and hesperidin.

In another preferred embodiment said composition is a food, food material, animal feed or pharmaceutical composition.

The figure shows:

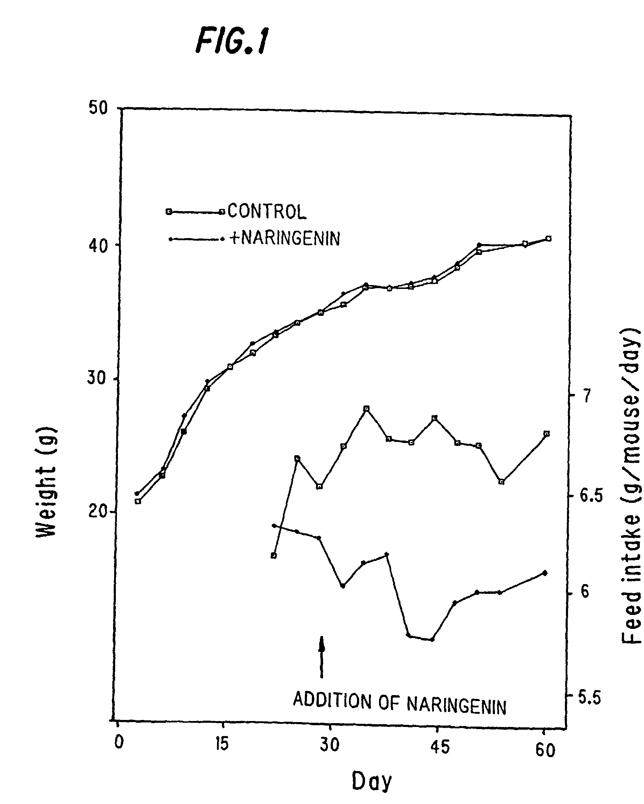

Fig. 1 is a graph showing the change in weight of diabetic mice and the intake of feed during breeding.

The flavanone used in the present invention is present mainly in citrus plants, namely plants belonging to Aurantioideae of Rutaceae in Rutales. Besides, it is also present in plants belonging to Compositae, Rosaceae and Labiatae. For example, when a citrus plant is used as a starting material, flavaonone can be found in the rind, the flesh, juice lees and the like.

Specific examples of the flavanone include naringenin, naringenin-7-glycoside, naringin, hesperetin and hesperidin. In the present invention, these can be used either singly or in combination.

The flavanone in the composition is used in an amount which is basically effective for cell differentiation into a fat cell. Specifically, this amount has to be determined in consideration of the use of said composition. For example, when the composition is used as a beverage, the amount of the flavanone has to be between 0.001 and 10% by weight. Further, when the composition is orally administered in the form of a solid, a powder, granules or a paste as a food, a food material or a drug, the flavanone may be contained in an amount of at least 0.001% by weight. The upper limit is 100% by weight, meaning that the flavanone may be used singly.

When the amount of the flavanone in the composition is less than the lower limit in any of the above-mentioned forms, no satisfactory activity of accelerating cell differentiation into a fat cell is provided.

A commercially available flavanone may be used. The flavanone can also be extracted from plants such as the above-mentioned citrus fruits and the like. The extraction may be conducted in a usual manner using an organic solvent such as methanol, ethanol, butanol or diethyl ether. For example, the extraction is conducted using a citrus (a mandarin orange) as a starting material as described below.

First, from 1 to 3 liters of an organic solvent such as methanol or the like are added to 1 kg of the rind of a citrus as a starting material, and the mixture is heat-refluxed, and extracted to obtain an extract. This extract is concentrated, and evaporated to dryness. To the resulting product are added 500 ml of an organic solvent such as butanol or the like and 500 ml of water, and these are stirred. Subsequently, the reaction product is allowed to stand to divide the same into an aqueous layer and an organic solvent layer.

Then, the layer of the organic solvent such as butanol or the like is concentrated, and evaporated to dryness. To this is added an organic solvent such as diethyl ether or the like, and these are stirred. Thereafter, the product is centrifuged, and ether is added to the resulting precipitate to remove any soluble substance. The thus-obtained ether-insoluble product (precipitate) is recovered as flavanone-containing fraction. Further, the product is fractionated into each ingredient by a usual method such as chromatography or the like as required. It can be verified through thin-layer chromatography or the like that the flavanone is contained therein.

The thus-extracted flavanone exhibits an activity of accelerating cell differentiation into a fat cell, and this effect can be increased by the co-treatment with insulin.

In the composition of the present invention, commercially available pure flavanone can be used. Also, a crude product or a purified product extracted from various plants can also be used as stated above. The composition can be prepared by adding suitable amounts of ordinary additives such as a filler, a stabilizer, an excipient and the like,if required. Depending on the use, the composition of the present invention is used in one of the above-mentioned various forms.

The composition for accelerating cell differentiation of the present invention is administered to the human body orally or through intravenous injection to differentiate a precursor fat cell into a fat cell and to activate sugar or lipid metabolism. Consequently, the sugar or lipid level in the blood is decreased in expectation of the improvement or the prevention of diseases such as diabetes, hyperlipemia and the like. Further, the composition can be applied to domestic animals to increase fat cells; this is effective for providing high-fat meat (marbled beef or the like). Accordingly, this composition can be used as feed additive.

Hormones and synthetic compounds are problematic with regard to side effects. However, in the composition for accelerating the cell differentiation of the present invention, a natural ingredient contained in plants such as citrus plants and the like is used as an active ingredient, and there is no fear at all of endangering the safety. Further, the present invention also provides a new method of utilizing the rind and the like of citrus fruits which were scarcely used so far. Accordingly, the present invention provides an environment-friendly technique.

The composition for accelerating the cell differentiation of the present invention acts to accelerate differentiation of a precursor fat cell into a fat cell and to activate the sugar or lipid metabolism thereby decreasing the sugar or lipid level in the blood.

Accordingly, it is possible to improve or prevent such diseases as diabates, hyperlipemia and the like by means of in vivo administration, like, for example, oral administration, intravenous injection, etc.

It is also possible to develop a feed since a high-fat meat (marbled beef or the like) can be provided by accelerating the differentiation into a fat cell and increasing fat tissue.

Further, the active ingredient of this composition is a natural food ingredient and is completely safe.

The examples illustrate the invention.

### Example 1

Three liters of methanol were added to 1 kg of citrus rind, heat-refluxed and extracted. The resulting extract was concentrated with a rotary evaporator, and evaporated to dryness. Then, 500 ml of water and 500 ml of n-butanol were added to the concentrate evaporated to dryness. The mixture was shaken, stirred, allowed to stand, and separated into an aqueous layer and a butanol layer. The butanol layer was evaporated to dryness under reduced pressure. Diethyl ether was added thereto, and the mixture was shaken, and stirred. Thereafter, the reaction product was centrifuged at 3,000 rpm for 10 minutes to remove any ether-soluble substance with the supernatant. Ether was further added to the resulting precipitate to remove any soluble substance. This procedure was repeated several times. The thus-obtained ether-insoluble substance was recovered as a flavanone-containing fraction.

### Example 2

The fraction obtained in Example 1 was subjected to reversed-phase HPLC (the gradient elution was conducted with an aqueous solution containing 0.1 % trifluoroacetic acid in which the concentration of acetonitrile varied within the range of from 0 to 50% using an ODS column, and the detection was carried out with absorbance of 262 nm) to obtain components, namely, naringenin, naringenin-7-glycoside, naringin, hesperetin and hesperidin.

### Example 3

Mouse precursor fat cells 3T3-L1 were incubated at 37°C in a DME medium (Dulbecco's modified Eagle medium) containing 10 % fetal bovine serum using a 6-well multi-plate. The medium was changed every 2 or 3 days. After the cells were gathered densely, the differentiation was induced. That is, naringenin or hesperetin was added to the medium such that its amount reached 1, 10, 30, 100 or 300 µM. In this manner, the differentiation was induced for 2 days. A medium free of flavanone was prepared as a control, and the differentiation was conducted in the same manner.

After the completion of the differentiation, the incubation was continued in the initial medium. Naringenin or hesperetin was continuously added when the medium was changed. After approximately 1 week, the amount of glycerol-3-phosphate dehydrogenase activity (hereinafter abbreviated as a GPDH activity") in the medium, which is an index of differentiation into fat cells, was measured. This enzyme is important in the sugar or lipid metabolism, and when a precursor fat cell is differentiated into a fat cell, the activity is increased. Accordingly, when this enzymatic activity is increased, it means that differentiation occured.The results are shown in Table 1.

**Table 1**

| Sample | | GPDH activity | |
|---|---|---|---|
| | | (NADH, nmol/mg protein) ^{a)} | (%) ^{b)} |
| Control | | 23.7 ± 2.7 | 100 |
| naringenin | 1 µM | 28.6 ± 3.1 | 121 |
| | 10 µM | 30.6 ± 2.2 | 129 |
| | 30 µM | 54.5 ± 3.5 | 230 |
| | 100 µM | 242.4 ± 35.3 | 1023 |
| | 300 µM | 614.0 ± 111.4 | 2591 |
| hesperetin | 1 µM | 28.0 ± 4.4 | 118 |
| | 10 µM | 28.9 ± 0.9 | 122 |
| | 30 µM | 30.7 ± 12.5 | 130 |
| | 100 µM | 249.4 ± 156.0 | 1052 |
| | 300 µM | 314.1 ± 21.9 | 1325 |

| | | | |
|---|---|---|---|
| a) average value± standard deviation | | | |
| b) value given when the value of the control is rated 100%. | | | |

As shown in Table 1, the GPDH activity of any sample containing naringenin or hesperetin was higher than that of the control. Clearly, the flavanones themselves had an effect of accelerating differentiation into a fat cell. Further, the acceleration of differentiation with the flavanones was increased depending on the concentrations thereof.

### Example 4

Example 3 was repeated except that insulin and various flavanones were added to the medium for the induction of differentiation. Even during incubation in DME medium after completion of the induction, insulin and flavanones were continuously added. A medium containing only insulin was used as a control. The concentration of insulin added to the medium was 1 µM in all cases. The results are shown in Table 2.

**Table 2**

| Sample | | GPDH activity (%) ^{a)} |
|---|---|---|
| control | | 100 |
| naringenin | 30 µM | 369 |
| | 100 µM | 2479 |
| naringenin-7-glycoside | 30 µM | 328 |
| | 100 µM | 2261 |
| naringin | 30 µM | 146 |
| hesperetin | 30 µM | 356 |
| | 100 µM | 529 |
| hesperidin | 30 µM | 184 |
| | 100 µM | 316 |

| | | |
|---|---|---|
| a) value given when the value of the control is rated 100%. | | |

### Example 5

The differentiation was induced as in Example 4 except that a flavanone-containing fraction extracted from the rind of citrus in

Example 1 was used as a differentiation accelerating substance instead of various flavanones. The results are shown in Table 3.

**Table 3**

| Sample | GPDH activity | |
|---|---|---|
| | (NADH, nmol/mg protein) ^{a}⁾ | (%) ^{b)} |
| control | 88.7 ± 4.5 | 100 |
| extract (100 µg/ml) | 2952.0 ± 167.0 | 3328 |

| | | |
|---|---|---|
| a) average value± standard deviation | | |
| b) value given when the value of the control is rated 100%. | | |

It could be demonstrated that even when the extract containing a plurality of the flavanones was added, the activity of accelerating the differentiation into a fat cell was exhibited as in the case of adding these substances singly.

### Example 6

An effect of accelerating glucose uptake with naringenin was examined by the following method. First, mouse precursor fat cells 3T3-L1 were incubated at 37°C in DME medium (Dulbecco's modified Eagle medium) containing 10 % fetal bovine serum using a 6-well multi-plate. The medium was changed every 2 or 3 days. After the cells were gathered densely, differentiation was induced. That is, naringenin was added to the medium such that its amount reached 100 or 300 µM. In this manner, the differentiation was induced for 10 days.

After 10 days, the cells differentiated were washed with Hanks' buffer, and retained in Hanks' buffer containing 1 µM insulin and 1 % bovine serum albumin at 37°C for 5 minutes. Thereafter, 0.2 µCi of radioactive glucose (made by NEN) were added thereto, and the mixture was maintained at 37°C for 30 minutes. The glucose uptake in cells was measured by extracting from the cells a neutral fat synthesized from radioactive glucose and measuring its radioactivity. Further, when a 1 % bovine serum albumin-containing Hanks' buffer free from insulin was used for washing the differentiated cells, the glucose uptake of cells was likewise measured. The results are shown in Table 4.

**Table 4**

| Sample | Addition of insulin (1 µM) | Glucose uptake ^{a)} (fmol/mg protein) | Effect of insulin | |
|---|---|---|---|---|
| | | | (presence-absence) ^{b)} | (%) ^{c)} |
| control | no | 1.11 ± 0.05 | 2.9 | 100 |
| | yes | 4.02 ± 0.16 | | |
| naringenin 100 µM | no | 4.40 ± 0.49 | 14.2 | 490 |
| | yes | 18.58 ± 0.31 | | |
| naringenin 300 µM | no | 7.34 ± 0.35 | 27.5 | 948 |
| | yes | 34.88 ± 0.27 | | |

| | | | | |
|---|---|---|---|---|
| a) average value ± standard deviation | | | | |
| b) difference in glucose uptake between the presence and the absence of insulin. | | | | |
| c) value given when the value of the control is rated 100%. | | | | |

As is clear from Table 4, the addition of insulin markedly activated the glucose uptake into cells, meaning that the sensitivity to insulin was increased.

### Example 7

The effect of accelerating the glucose uptake with various flavanones was examined as in Example 6. A medium free from various flavanones was used as a control. The results are shown in Table 5.

**Table 5**

| Sample | Addition of insulin (1 µM) | Glucose uptake^{a)} (fmol/mg protein) | Effect of insulin (presence-absence)^{b)} | (%)^{c)} |
|---|---|---|---|---|
| control | no | 2.78 ± 0.10 | 6.8 | 100 |
| | yes | 9.48 ± 0.89 | | |
| naringenin | no | 4.21 ± 0.33 | 18.5 | 272 |
| 100 µM | yes | 22.78 ± 1.20 | | |
| naringin | no | 3.18 ± 0.50 | 9.2 | 134 |
| 100 µM | yes | 12.31 ± 1.15 | | |
| hesperetin | no | 4.91 ± 0.22 | 11.1 | 163 |
| 100 µM | yes | 16.04 ± 0.35 | | |
| hesperidin | no | 3.26 ± 0.10 | 10.8 | 156 |
| 100 µM | yes | 14.07 ± 1.37 | | |

| | | | | |
|---|---|---|---|---|
| a) average value± standard deviation | | | | |
| b) difference in glucose uptake between the presence and the absence of insulin. | | | | |
| c) value given when the value of the control is rated 100%. | | | | |

As shown in Table 5, the glucose uptake in the cells treated with the flavanones was activated through the addition of insulin. Further, the glucose uptake was most activated with naringenin among the four types of flavanones in the test.

### Example 8

In order to examine the activity of increasing the lipoprotein lipase activity with naringenin, the following test was conducted. First, mouse precursor fat cells 3T3-L1 were incubated at 37°C in DME medium (Dulbecco's modified Eagle medium) containing 10 % fetal bovine serum using a 6-well multi-plate. The medium was changed every 2 or 3 days. After the cells were gathered densely, differentiation was induced. That is, naringenin was added to the medium such that its amount reached 100 or 300 µM. In this manner, the differentiation was induced for 10 days.

After 10 days, the lipoprotein lipase activity in the medium was measured by extracting radioactive oleic acid isolated from radioactive triolein (made by NEN), and determining the radioactivity thereof.

Lipoprotein lipase is an enzyme that participates in the decomposition of the neutral fat in the blood, and it is said that insulin increases said enzymatic activity in the fat cell. Accordingly, when this enzymatic activity is increased, the neutral fat in the blood is actively decomposed and reduced. The amount of insulin added to the medium is 1 µM in every case. The results are shown in Table 6. The absence of naringenin is used as a control.

**Table 6**

| Sample | | Lipoprotein lipase activity | |
|---|---|---|---|
| | | (oleic acid, nmol/ml/min) | (%) ^{a)} |
| control | | 2.71 ± 0.60 | 100 |
| naringenin | 1 µM | 5.05 ± 0.33 | 186 |
| | 10 µM | 5.22 ± 0.37 | 193 |
| | 30 µM | 7.68 ± 0.57 | 283 |
| | 100 µM | 5.46 ± 0.81 | 201 |

| | | | |
|---|---|---|---|
| a) value given when the value of the control is rated 100%. | | | |

When naringenin was added, lipoprotein lipase activity was markedly increased independently from the amount added as compared with the control, and it was found that naringenin had itself the activity of increasing lipoprotein lipase activity. Further, lipoprotein lipase activity was highest when the amount of naringenin was 30 µM.

### Example 9

An influence of naringenin on the serum glucose concentration was examined using KK-A^{y} mice which are diabetic mice.

First, 12 four-week-old KK-A^{y} mice (weight approximately 20 g) were grown for 28 days at a temperature of 23°C by freely feeding feed (trade name: Oriental Yeast MF) and water to them. These mice were divided into two groups, namely, a naringenin addition group and a control group on the 29th day, and were further grown for 60 days. That is, the feed containing 1 % naringenin was fed to the mice in the naringenin addition group, and the feed free from naringenin to the mice in the control group, respectively.

On the 60th day, the serum glucose concentration at the time of fasting was measured by colorimetry. The relationship of the weight change and the feed intake during that period is shown in Fig. 1, and the results of the serum glucose concentration in Table 7, respectively.

In Fig. 1. ◆ shows the naringenin addition group, and □ shows the control group.

**Table 7**

| | Serum glucose concentration (mg/dl) ^{a)} |
|---|---|
| Control group | 257.3 ± 56.8 |
| Naringenin addition group | 186.7 ± 34.6^{b)} |

| | |
|---|---|
| a) average value ± standard deviation | |
| b) significantly decreased as compared with the control group (p = 0.014) | |

As is clear from Fig. 1, in the naringenin addition group, although the intake of feed was decreased by approximately 1 g/day for a mouse, the weight was continuously increased, and there was no difference at all between the naringenin and the control group, meaning that the mice grew normally even with the addition of naringenin.

Further, the serum glucose concentration of the naringenin addition group was significantly decreased as compared with that of the control group as shown in Table 7.

The above-mentioned results demonstrate that naringenin effectively acts to improve or prevent diabetes by decreasing the glucose concentration in the blood.

## Claims

1. A composition for accelerating cell differentiation into a fat cell comprising a flavanone and insulin in an amount effective for accelerating cell differentiation into a fat cell.

2. The composition of claim 1, which is a food, food material or animal feed.

3. A pharmaceutical composition comprising a flavanone and insulin and, optionally, a pharmaceutically acceptable carrier.

4. The composition of claim 1 or 2, or the pharmaceutical composition of claim 3, wherein said flavanone is at least one compound selected from the group consisting of naringenin, naringenin-7-glycoside, naringin, hesperetin and hesperidin.

5. Use of a flavanone and insulin for the preparation of a composition for accelerating cell differentiation into a fat cell.

6. Use of a flavanone and insulin for the preparation of a composition for decreasing blood sugar or lipid level.

7. Use of a flavanone and insulin for the preparation of a composition for treating or preventing diabetes, hyperlipemia, hypertension, gout, ischemic heart disease, fatty liver, cholelithiasis, menstrual disorders or sterility.

8. The use of any one of claims 5 to 7, wherein said flavanone is at least one compound selected from the group consisting of naringenin, naringenin-7-glycoside, naringin, hesperetin and hesperidin.

9. The use of any one of claims 5 to 8, wherein said composition is a food, food material, animal feed or pharmaceutical composition.

## Patentansprüche

1. Zusammensetzung zur Beschleunigung von Zelldifferenzierung in eine Fettzelle, umfassend ein Flavanon und Insulin in einer Menge, die wirksam ist zur Beschleunigung von Zelldifferenzierung in eine Fettzelle.

2. Zusammensetzung nach Anspruch 1, welche ein Nahrungsmittel, Nahrungsmittelmaterial oder Tierfutter ist.

3. Arzneimittel, umfassend ein Flavanon und Insulin und, gegebenenfalls, einen pharmazeutisch verträglichen Träger.

4. Zusammensetzung nach Anspruch 1 oder 2 oder Arzneimittel nach Anspruch 3, wobei das Flavanon zumindest eine Verbindung ist, ausgewählt aus der Gruppe bestehend aus Naringenin, Naringenin-7-glycosid, Naringin, Hesperetin und Hesperidin.

5. Verwendung eines Flavanons und Insulins zur Herstellung einer Zusammensetzung zur Beschleunigung von Zelldifferenzierung in eine Fettzelle.

6. Verwendung eines Flavanons und Insulins zur Herstellung einer Zusammensetzung zur Absenkung des Blutzucker- oder Blutfettspiegels.

7. Verwendung eines Flavanons und Insulins zur Herstellung einer Zusammensetzung zur Behandlung oder Vorbeugung von Diabetes, Hyperlipämie, Bluthochdruck, Gicht, ischämischer Herzkrankheit, Fettleber, Cholelithiasis, Menstruationsstörungen oder Sterilität.

8. Verwendung nach einem der Ansprüche 5 bis 7, wobei das Flavanon zumindest eine Verbindung ist, ausgewählt aus der Gruppe bestehend aus Naringenin, Naringenin-7-glycosid, Naringin, Hesperetin und Hesperidin.

9. Verwendung nach einem der Ansprüche 5 bis 8, wobei die Zusammensetzung ein Nahrungsmittel, Nahrungsmittelmaterial, Tierfutter oder ein Arzneimittel ist.

## Revendications

1. Composition pour accélérer la différenciation cellulaire en adipocyte comprenant une flavanone et de l'insuline en une quantité efficace pour accélérer la différenciation cellulaire en adipocyte.

2. Composition selon la revendication 1, qui est un aliment, une denrée alimentaire ou un aliment pour animaux.

3. Composition pharmaceutique comprenant une flavanone et de l'insuline et, le cas échéant, un véhicule pharmaceutiquement acceptable.

4. Composition selon la revendication 1 ou 2, ou composition pharmaceutique selon la revendication 3, dans laquelle ladite flavanone est au moins un composé choisi dans le groupe constitué par la naringénine, le naringénine-7-glycoside, la naringine, l'hespérétine et l'hespéridine.

5. Utilisation d'une flavanone et d'insuline pour la préparation d'une composition destinée à accélérer la différenciation cellulaire en adipocyte.

6. Utilisation d'une flavanone et d'insuline pour la préparation d'une composition destinée à abaisser le taux de sucre ou de lipide dans le sang.

7. Utilisation d'une flavanone et d'insuline pour la préparation d'une composition destinée à traiter ou prévenir le diabète, l'hyperlipémie, l'hypertension, la goutte, la cardiopathie ischémique, la stéatose hépatique, la lithiase biliaire, les troubles menstruels ou la stérilité.

8. Utilisation selon l'une quelconque des revendications 5 à 7, dans laquelle ladite flavanone est au moins un composé choisi dans le groupe constitué par la naringénine, le naringénine-7-glycoside, la naringine, l'hespérétine et l'hespéridine.

9. Utilisation selon l'une quelconque des revendications 5 à 8, dans laquelle ladite composition est un aliment, une denrée alimentaire, un aliment pour animaux ou une composition pharmaceutique.
